# EUROPEAN PATENT SPECIFICATION

(11) **EP 3 681 854 B1**
(45) Date of publication and mention of the grant of the patent: **29.01.2025**
(21) Application number: 18856886.9
(22) Date of filing: 13.09.2018
(51) Int. Cl.: C07C 67/03, C08L 101/00, C07C 29/128, C07C 67/08

(54) **TEREPHTHALIC ACID ESTERS FORMATION**
FORMUNG VON TEREPHTHALSÄUREESTERN
FORMATION D'ESTERS D'ACIDE TÉRÉPHTALIQUE

(30) Priority: 15.09.2017 US 201715706484
(43) Date of publication of application: 22.07.2020
(60) Divisional application: 24221207.4
(73) Proprietor: 9449710 Canada Inc., Terrebonne, Québec J6Y 1Y4 (CA)
(72) Inventor: ESSADDAM, Adel, Terrebonne, Québec J6Y 1Y4 (CA); ESSADDAM, Fares, Terrebonne, Québec J6Y 1Y4 (CA)
(74) Representative: Mewburn Ellis LLP
(86) International application number: PCT/CA2018/051135
(87) International publication number: WO 2019/051597

(56) References cited:
- CA-A1- 2 069 500
- JP-A- 2001 192 492
- US-A- 3 501 420
- US-A1- 2017 008 826
- US-A1- 2017 113 995
- MOHSIN M. A. ET AL: "Sodium Methoxide Catalyzed Depolymerization of Waste Polyethylene Terephthalate under Microwave Irradiation", KATALIZ V PROMYSHLENNOSTI, vol. 17, no. 4, 1 January 2017 (2017-01-01), RU, pages 278 - 286, XP055796953, ISSN: 1816-0387, Retrieved from the Internet <URL:http://www.catalysis-kalvis.ru/jour/article/viewFile/421/381> DOI: 10.18412/1816-0387-2017-4-278-286
- NAMBOORI C G G ET AL: "Steric Effects in the Basic Hydrolysis of Poly(ethy1ene Terephthalate)", JOURNAL OF APPLIED POLYMER SCIENCE, 1 January 1968 (1968-01-01), pages 1999 - 2005, XP055797459, Retrieved from the Internet <URL:https://onlinelibrary.wiley.com/doi/epdf/10.1002/app.1968.070120901> [retrieved on 20210421]
- KUROKAWA ET AL.: "Methanolysis of polyethylene terephthalate (PET) in the presence of aluminium triisopropoxide catalyst to form dimethyl terephthalate and ethylene glycol", POLYMER DEGRADATION AND STABILITY, vol. 79, 2003, pages 529 - 533, XP004415928, DOI: doi:10.1016/S0141-3910(02)00370-1

## Description

### FIELD OF THE INVENTION

The present disclosure relates to the formation of ester derivatives from polyesters and more specifically to the formation of terephthalic acid esters from polyethylene terephthalate (PET) or poly(ethylene glycol-co-1,4-cyclohexanedimethanol terephthalate) (PETG). The present disclosure also relates to the formation of dimethyl terephthalate (DMT). The present disclosure also relates to the formation of monoethylene glycol (MEG).

### BACKGROUND OF THE INVENTION

The polyethylene terephthalate (PET) bottle resin market has been growing strongly as PET resins have replaced glass in carbonated soft drink, bottled water and food containers.

Dimethyl terephthalate (DMT) is primarily used in the manufacture of polyethylene terephthalate (PET) for fiber, film, container plastics, and specialty plastics applications.

The largest polyester sector is the fibers market where it is used to make clothes, home textiles such as sheets and curtains, carpets and rugs, and industrial products such as tire cord, seat belts, hoses and ropes. PET film is utilized in electrical applications such as dielectric metal foil capacitors and for food packaging.

The growth in polyester has not been converted into DMT demand. For most grades of polyester used in textiles and food and beverage containers, it is more economical to use purified terephthalic acid rather than DMT.

Mohsin et al. Sodium Methoxide Catalyzed Depolymerization of Waste Polyethylene Terephthalate Under Microwave Irradiation, KATALIZ V PROMYSHLENNOSTI, vol. 17, no. 4, 1 January 2017, pages 278-286 describes the chemical recycling of polyethylene terephthalate to produce terephthalic acid using in situ hydrolysis with sodium methoxide in methanol and dimethyl sulfoxide as solvent under microwave irradiation.

US 2017/008826 A1 relates to the depolymerization of polyethylene terephthalate and the recovery of terephthalic acid and ethylene glycol.

Namboori et al., Steric Effects in the Basic Hydrolysis of Poly(ethylene Terephthalate), JOURNAL OF APPLIED POLYMER SCIENCE, 1 January 1968, pages 1999-2005 describes a comparative study of the reactivity of hydroxide and various alkoxide anions made by determining the percentage weight loss of poly (ethylene terephthalate) fiber.

JP 2001 192492 A describes a method for refining thermoplastic polyester resin comprising brining a crushed material of thermoplastic polyester resin waste into contact with a mixed solution of an alkali with an alcohol and removing 0.5-15 wt.% of the total amount of the crushed material as impurities and then recovering the crushed material of the refined resin.

Kurokawa et al., Methanolysis of polyethylene terephthalate (PET) in the presence of aluminium triisopropoxide catalyst to form dimethyl terephthalate and ethylene glycol, Polymer Degradation and Stability, vol. 79, 2003, pages 529-533, describes aluminium triisopropoxide promoted methanolyysis of polyethylene terephthalate to form monomers, dimethyl terepthathalate and ethylene glycol in an equimolar ratio.

US 3 501 420 A describes the production of high purity polyester depolymerization products form polyester scrap materials. In particular, the production of substantially colourless solutions of difunctional dicarboxylic acids and difunctional glycols from dye- or other impurity-containing waste polyester, which acids and glycols may be employed I the production of high quality polyester products.

US 2017/113995 A1 relates to a process for producing a terephthalate ester comprising providing reclaimed carpet material comprising a polyester component and reacting the polyester component with an alcohol.

### SUMMARY OF THE INVENTION

Disclosed herein is a process for the transformation of a polyester selected from the group consisting of polyethylene terephthalate and polyethylene glycol-co-1,4-cyclohexanedimethanol terephthalate) into a terephthalate of Formula (I): 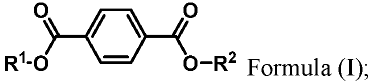
wherein R¹ and R² are independently selected from the group consisting of hydrogen, C₁-C₆ alkyl, C₁-C₆ haloalkyl, C₁-C₆ hydroxyalkyl, C₃-C₈ cycloalkyl, (C₁-C₆ alkyl)(C₃-C₈ cycloalkyl), aryl, and (C₁-C₆ alkyl)(aryl); provided that one of R¹ or R² is not hydrogen;
wherein C₃-C₈ cycloalkyl is optionally substituted with oxo, halogen, methyl, ethyl, -CN, -CF₃, -OH, -OMe, -NH₂, or -NO₂;
wherein C₁-C₆ alkyl is optionally substituted with oxo, halogen, -CN, -CF₃, -OH, -OMe, -NH₂, or -NO₂; and
wherein aryl is optionally substituted with halogen, amino, nitrile, nitro, hydroxyl, alkyl, alkenyl, alkynyl, haloalkyl, alkoxy, aryl, cycloalkyl, heterocycloalkyl, heteroaryl, -CN, -CF₃, or -OMe,
wherein cycloalkyl in the definitions above refers to a partially or fully saturated, monocyclic or polycyclic carbocyclic ring;
the process comprising pre-treating the polyester with a solvent for swelling the polyester, and admixing the polyester with a mixture comprising:
   (a) an alcoholic solvent; and
   (b) a sub-stoichiometric amount of an alkoxide
wherein the process is performed at a temperature between 25 °C and 100 °C;
wherein the ratio of the polyester to the alkoxide is between 20:1 and 125:1 (w:w); and
wherein the pre-treating the polyester with the solvent for swelling the polyester is performed for between 5 mins and 60 mins prior to admixing the polyester with the mixture comprising (a) and (b).

The process for the transformation of a polyester is in accordance independent claim 1. Preferred embodiments are disclosed in the dependent claims.

In some embodiment of the process, R¹ or R² is methyl.

In some embodiment of the process, the alcoholic solvent is selected from the group consisting of methanol, ethanol, n-propanol, isopropanol, t-butanol, ethylene glycol, glycerol, cyclohexane-1,4-diyldimethanol, phenol, benzyl alcohol, and any combinations thereof.

In some embodiment of the process, the alcoholic solvent is methanol.

In some embodiment of the process, the solvent for swelling the polyester is selected from the group consisting of a non polar solvent, a polar aprotic solvent, a polar protic solvent, and any combinations thereof.

In some embodiment of the process, the solvent for swelling the polyester is selected from the group consisting of DMSO, DMF, acetone, a halogenated solvent, n-hexane, nitrobenzene, methanol, benzyl alcohol, benzaldehyde, and any combinations thereof.

In some embodiment of the process, the solvent for swelling the polyester is a halogenated solvent.

In some embodiment of the process, the solvent for swelling the polyester is a methanol.

In some embodiment of the process, the ratio of solvent for swelling the polyester to alcoholic solvent is between 0.1: 1 and 2:1 (w:w).

In some embodiment of the process, the ratio of solvent for swelling the polyester to alcoholic solvent is between 0.5:1 and 1:1 (w:w).

In some embodiment of the process, the alkoxide is selected from the group consisting of an alkali metal alkoxide, an alkaline earth metal alkoxide, a metal alkoxide, an ammonium alkoxide and any combinations thereof.

In some embodiment of the process, the alkoxide is selected from the group consisting of sodium methoxide, potassium ethoxide, aluminum tri-n-propoxide, and tetrabutylammonium methoxide.

In some embodiment of the process, the alkoxide is sodium methoxide.

In some embodiment of the process, the alkoxide is generated in-situ by addition of an alkali metal, an alkaline earth metal, or a metal to the alcoholic solvent.

In some embodiment of the process, the ratio of polyester to alkoxide is between 15:1 and 125:1 (w:w).

In some embodiment of the process, the ratio of polyester to alkoxide is between 20:1 and 25:1 (w:w).

In some embodiment of the process, the ratio of polyester to alkoxide is between 15:1 and 60:1 (w:w).

In some embodiment of the process, the ratio of polyester to alkoxide is between 20:1 and 50:1 (w:w).

In some embodiment of the process, the process is performed until 80% yield of the terephthalate of Formula (I) is achieved.

In some embodiment of the process, the process is performed without external heat.

In some embodiment of the process, the process is performed at a temperature between 25 °C and 85 °C.

In some embodiment of the process, the process is performed at a temperature between 25 °C and 80 °C.

In some embodiment of the process, the process is performed at atmospheric pressure.

In some embodiment of the process, the polyester is polyethylene terephthalate.

In some embodiment of the process, the process further comprises recovering monoethylene glycol (MEG).

In some embodiment of the process, the polyester is in the form of polymer flakes and is part of a feedstock.

In some embodiment of the process, the average polymer flakes particle size is between 1 mm and 20 mm.

In some embodiment of the process, the average polymer flakes particle size is between 1 mm and 5 mm.

Also disclosed herein is a process for the transformation of polyethylene terephthalate into dimethyl terephthalate, comprising pre-treating the polyethylene terephthalate with a solvent for swelling the polyethylene terephthalate, and admixing the polyethylene terephthalate with a mixture comprising:
(a) methanol; and
(b) a sub-stoichiometric amount of an alkoxide.

In some embodiment of the process, the solvent for swelling the polyethylene terephthalate is selected from the group consisting of a halogenated solvent, DMSO, benzyl alcohol, methanol, and any combinations thereof.

In some embodiment of the process, the solvent for swelling the polyethylene terephthalate is a halogenated solvent.

In some embodiment of the process, the solvent for swelling the polyethylene terephthalate is methanol.

In some embodiment of the process, the ratio of polyethylene terephthalate to alkoxide is between 20:1 and 25:1 (w:w).

In some embodiment of the process, the ratio of polyester to alkoxide is between 20:1 and 50:1 (w:w).

In some embodiment of the process, the alkoxide is selected from the group consisting of sodium methoxide, potassium ethoxide, aluminum tri-n-propoxide, and tetrabutylammonium methoxide.

In some embodiment of the process, the alkoxide is sodium methoxide.

In some embodiment of the process, the ratio of solvent for swelling the polyethylene terephthalate to methanol is between 0.5:1 and 1:1 (w:w).

In some embodiment of the process, the process is performed until 80% yield of dimethyl terephthalate is achieved.

In some embodiment of the process, the process is performed without external heat.

In some embodiment of the process, the process is performed at a temperature between 25 °C and 85 °C.

In some embodiment of the process, the process is performed at a temperature between 25 °C and 80 °C.

In some embodiment of the process, the process is performed at atmospheric pressure.

In some embodiment of the process, the process further comprises recovering monoethylene glycol (MEG).

In some embodiment of the process, the polyethylene terephthalate is in the form of polymer flakes and is part of a feedstock.

In some embodiment of the process, the average polymer flakes particle size is between 1 mm and 20 mm.

In some embodiment of the process, the average polymer flakes particle size is between 1 mm and 5 mm.

Also provided herein is dimethyl terephthalate (DMT) prepared using a process described herein.

Also provided herein monoethylene glycol (MEG) prepared using a process described herein.

Also provided herein is a reaction mixture comprising:
(a) a polyester selected from polyethylene terephthalate and polyethylene glycol-co-1,4-cyclohexanedimethanol terephthalate);
(b) a solvent for swelling the polyester;
(c) an alcoholic solvent; and
(d) a sub-stoichiometric amount of an alkoxide.

In some embodiment of a reaction mixture, the alcoholic solvent is selected from the group consisting of methanol, ethanol, n-propanol, isopropanol, t-butanol, ethylene glycol, glycerol, cyclohexane-1,4-diyldimethanol, phenol, benzyl alcohol, and any combinations thereof.

In some embodiment of a reaction mixture, the solvent for swelling the polyester is selected from the group consisting of a non polar solvent, a polar aprotic solvent, a polar protic solvent, and any combinations thereof.

In some embodiment of a reaction mixture, the solvent for swelling the polyester is a halogenated solvent.

In some embodiment of a reaction mixture, the solvent for swelling the polyester is methanol.

In some embodiment of a reaction mixture, the alcoholic solvent is methanol.

In some embodiment of a reaction mixture, the ratio of solvent for swelling the polyester to alcoholic solvent is between 0.5:1 and 1:1 (w:w).

In some embodiment of a reaction mixture, the ratio of polyethylene terephthalate to alkoxide is between 20:1 and 25:1 (w:w).

In some embodiment of a reaction mixture, the ratio of polyester to alkoxide is between 20:1 and 50:1 (w:w).

In some embodiment of a reaction mixture, the alkoxide is selected from the group consisting of sodium methoxide, potassium ethoxide, aluminum tri-n-propoxide, and tetrabutylammonium methoxide.

In some embodiment of a reaction mixture, the alkoxide is sodium methoxide.

In some embodiment of a reaction mixture, the polyester is polyethylene terephthalate.

### DETAILED DESCRIPTION OF THE INVENTION

Dimethyl terephthalate (DMT) is used in the production of polyesters, including polyethylene terephthalate (PET), polytrimethylene terephthalate (PTT), and polybutylene terephthalate (PBT). Because DMT is volatile, it is an intermediate in some schemes for the recycling of PET, e.g. from plastic bottles. Hydrogenation of DMT affords the diol 1, 4-cyclohexanedimethanol, which is a useful monomer in the formation of polyester resins.

DMT has been produced in a number of ways. Conventionally and still of commercial value is the direct esterification of terephthalic acid. Alternatively, it is prepared by alternating oxidation and methyl-esterification steps from para-xylene via methyl para-toluate. The method for the production of DMT from para-xylene and methanol consists of four major steps: oxidation, esterification, distillation, and crystallization. A mixture of para-xylene and pare-toluic ester is oxidized with air in the presence of a transition metal catalyst (Co/Mn). The acid mixture resulting from the oxidation is esterified with methanol to produce a mixture of esters. The crude ester mixture is distilled to remove all the heavy boilers and residue produced; the lighter esters are recycled to the oxidation section. The raw DMT is then crystallized to remove DMT isomers, residual acids, and aromatic aldehydes.

An Improvement in DMT production from PET recycling: due to the growing use of PET and PETG in the packaging and fiber (carpet and other textile) industries there is a need for an efficient, low energy, high yielding, and cost effective way to form DMT from PET or PETG.

### POLYESTERS

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

In some embodiments, the polyester is selected from polyethylene terephthalate (PET), polyethylene glycol-co-1,4-cyclohexanedimethanol terephthalate) (PETG), polyglycolide or polyglycolic acid (PGA), polylactic acid (PLA), polycaprolactone (PCL), polyhydroxybutyrate (PHB), polyethylene adipate (PEA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), Vectran^{®}, cutin, and any combinations thereof.

In some embodiments, the polyester is polyethylene terephthalate (PET):

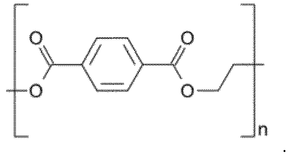

In some embodiments, the polyester is a terephthalic acid/ethylene glycol oligomer.

In some embodiments, the polyester is polyethylene glycol-co-1,4-cyclohexanedimethanol terephthalate) (PETG):

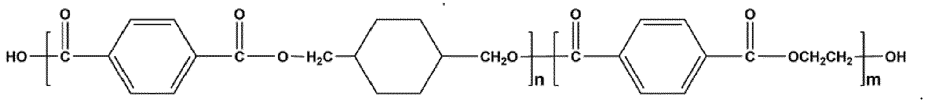

In some embodiments, the polyester is polyglycolide or polyglycolic acid (PGA),

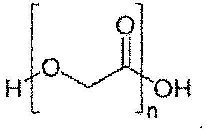

In some embodiments, the polyester is polylactic acid (PLA):

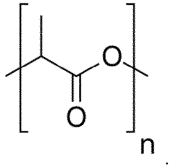

In some embodiments, the polyester is polycaprolactone (PCL):

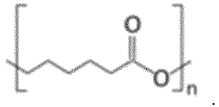

In some embodiments, the polyester is polyhydroxybutyrate (PHB):

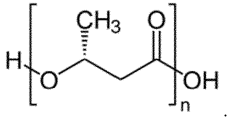

In some embodiments, the polyester is polyethylene adipate (PEA):

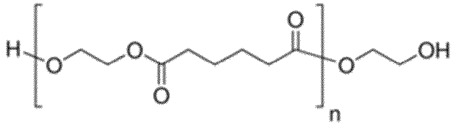

In some embodiments, the polyester is polybutylene succinate (PBS):

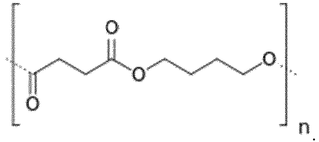

In some embodiments, the polyester is poly(3-hydroxybutyrate-co-3-hydroxyvalerate) (PHBV):

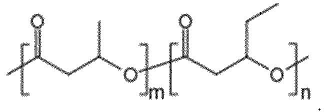

In some embodiments, the polyester is polybutylene terephthalate (PBT):

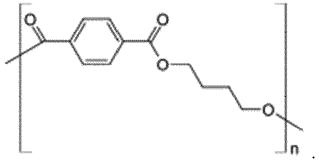

In some embodiments, the polyester is polytrimethylene terephthalate (PTT):

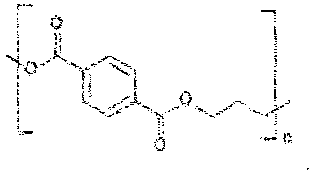

In some embodiments, the polyester is polyethylene naphthalate (PEN):

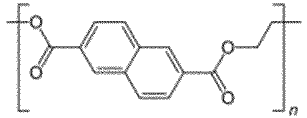

In some embodiments, the polyester is Vectran^{®}:

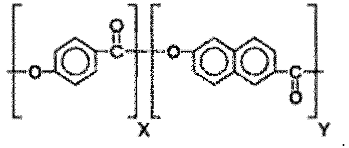

In some embodiments, the polyester is cutin. Cutin is one of two waxy polymers that are the main components of the plant cuticle, which covers all aerial surfaces of plants. Cutin consists of omega hydroxy acids and their derivatives, which are interlinked via ester bonds, forming a polyester polymer. There are two major monomer families of cutin, the C16 and C18 families. The C16 family consists mainly of 16-hydroxy palmitic acid and 9,16- or 10,16-dihydroxypalmitic acid. The C18 family consists mainly of 18-hydroxy oleic acid, 9,10-epoxy-18-hydroxy stearic acid, and 9,10,18-trihydroxystearate. Tomato cutin consists of 16-hydroxy palmitic acid and 10,16-dihydroxypalmitic acid where the 10-isomer is largely dominant. The tomato cutin is a polyester biopolymer interesterificated. The significant proportion of secondary esters (esterification in the C-10 secondary hydroxyl) shows that the polyester structure is significantly branched.

In some embodiments, the polyester is in a feedstock comprising contaminants such as additional polymers (for example polyethylene, polypropylene, polyvinyl chloride (PVC), paper, colorants, dirt, ethylene vinyl alcohol (EvOH), polycarbonate (PC), polyvinylidene chloride (PVDC), ethylene vinyl acetate (EVA), glue, polyamide, or any combination thereof. In some embodiments, the feedstock comprises between 5% and 20% contaminants.

Mishra et al. (Kinetic and thermodynamic study of methanolysis of polyethylene terephthalate) waste powder Polym. Int. 52:337-342 (2003)) have shown that increase in the particle size of PET flakes causes a decrease in depolymerization. Mishra et al. recorded 127.5µm as the optimal particle size. There is a need for a depolymerization process that allows for the use of bigger polymer flakes. In some embodiments the feedstock comprises polymer flakes. In some embodiments, the average polymer flakes particle size is between 1 mm and 20 mm. In some embodiments, the average polymer flakes particle size is between 1 mm and 15 mm. In some embodiments, the average flakes particle size is between 1 mm and 10 mm. In some embodiments, the average polymer flakes particle size is between 1 mm and 5 mm.

### ESTER DERIVATIVES

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

In some embodiments, the polyester is polyglycolide or polyglycolic acid (PGA) and the ester derivative is a 2-hydroxyacetate derivative. In some embodiments, the ester derivative is methyl 2-hydroxyacetate.

In some embodiments, the polyester is polylactic acid (PLA) and the ester derivative is a 2-hydroxypropanoate derivative. In some embodiments, the ester derivative is methyl 2-hydroxypropanoate.

In some embodiments, the polyester is polycaprolactone (PCL) and the ester derivative is a 6-hydroxyhexanoate derivative. In some embodiments, the ester derivative is a methyl 6-hydroxyhexanoate.

In some embodiments, the polyester is polyhydroxybutyrate (PHB) and the ester derivative is a hydroxybutyrate derivative. In some embodiments, the ester derivative is methyl hydroxybutyrate.

In some embodiments, the polyester is polyethylene adipate (PEA) and the ester derivative is an adipate derivative. In some embodiments, the ester derivative is dimethyl adipate.

In some embodiments, the polyester is polybutylene succinate (PBS) and the ester derivative is a succinate derivative. In some embodiments, the ester derivative is dimethyl succinate.

In some embodiments, the polyester is poly(3-hydroxybutyrate-co-3-hydroxyvalerate) (PHBV) and the ester derivative is a hydroxybutyrate derivative, a hydroxyvalerate derivative, or a combination thereof. In some embodiments, the ester derivative is methyl hydroxybutyrate, methyl hydroxyvalerate, or a combination thereof.

In some embodiments, the polyester is polyethylene naphthalate (PEN) and the ester derivative is a naphthalate derivative. In some embodiments, the ester derivative is dimethyl naphthalate.

In some embodiments, the polyester is vectran and the ester derivative is a naphthoate derivative, a benzoate derivative, or a combination thereof. In some embodiments, the ester derivative is methyl hydroxynaphthoate or methyl hydroxybenzoate.

In some embodiments, the polyester is cutin and the ester derivative is a hydroxypalmitate or a dihydroxypalmitate derivative. In some embodiments, the ester derivative is methyl hydroxypalmitate or methyl dihydroxypalmitate.

In some embodiments, the polyester is polyethylene terephthalate (PET), polyethylene glycol-co-1,4-cyclohexanedimethanol terephthalate) (PETG), polytrimethylene terephthalate (PTT), or polybutylene terephthalate (PBT) and the ester derivative is a terephthalate derivative. In some embodiments, the terephthalate derivative is a compound of formula (I): 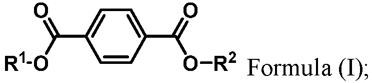 wherein R¹ and R² are independently selected from hydrogen, C₁-C₆ alkyl, C₁-C₆ haloalkyl, C₁-C₆ hydroxyalkyl, optionally substituted C₃-C₈ cycloalkyl, optionally substituted (C₁-C₆ alkyl)(C₃-C₈ cycloalkyl), optionally substituted aryl, and optionally substituted (C₁-C₆ alkyl)(aryl); provided that one of R¹ or R² is not hydrogen.

In some embodiments of a compound of Formula (I), R¹ and R² are independently C₁-C₆ alkyl or C₁-C₆ hydroxyalkyl.

In some embodiments of a compound of Formula (I), R¹ and R² are independently C₁-C₆ alkyl. In some embodiments of a compound of Formula (I), R¹ and R² are independently methyl, ethyl, propyl, isopropyl, or butyl. In some embodiments of a compound of Formula (I), R¹ and R² are independently methyl or ethyl. In some embodiments of a compound of Formula (I), R¹ and R² are methyl. In some embodiments of a compound of Formula (I), R¹ and R² are ethyl. In some embodiments of a compound of Formula (I), R¹ and R² are not hydrogen.

In some embodiments of a compound of Formula (I), R¹ and R² are independently C₁-C₆ hydroxyalkyl. In some embodiments of a compound of Formula (I), R¹ and R² are independently hydroxyethyl or propanediol. In some embodiments of a compound of Formula (I), R¹ and R² are hydroxyethyl. In some embodiments of a compound of Formula (I), R¹ and R² are 2-hydroxyethyl. In some embodiments of a compound of Formula (I), R¹ and R² are dihydroxypropyl. In some embodiments of a compound of Formula (I), R¹ and R² are 2,3-dihydroxypropyl.

In some embodiments of a compound of Formula (I), R¹ and R² are independently optionally substituted (C₁-C₆ alkyl)(C₃-C₈ cycloalkyl). In some embodiments of a compound of Formula (I), R¹ and R² are independently substituted (C₁-C₆ alkyl)(C₃-C₈ cycloalkyl). In some embodiments of a compound of Formula (I), R¹ and R² are 4-(hydroxymethyl)cyclohexyl)methyl.

In some embodiments of a compound of Formula (I), R¹ and R² are independently optionally substituted aryl. In some embodiments of a compound of Formula (I), R¹ and R² are phenyl.

In some embodiments of a compound of Formula (I), R¹ and R² are independently optionally substituted (C₁-C₆ alkyl)(aryl). In some embodiments of a compound of Formula (I), R¹ and R² are benzyl.

In some embodiments, the ester derivative contains less than 10% impurity (w/w). In some embodiments, the ester derivative contains less than 9% impurity (w/w). In some embodiments, the ester derivative contains less than 8% impurity (w/w). In some embodiments, the ester derivative contains less than 7% impurity (w/w). In some embodiments, the ester derivative contains less than 6% impurity (w/w). In some embodiments, the ester derivative contains less than 5% impurity (w/w). In some embodiments, the ester derivative contains less than 4% impurity (w/w). In some embodiments, the ester derivative contains less than 3% impurity (w/w). In some embodiments, the ester derivative contains less than 2% impurity (w/w). In some embodiments, the ester derivative contains less than 1% impurity (w/w). In some embodiments, the ester derivative contains less than 0.5% impurity (w/w). In some embodiments, the ester derivative contains less than 0.4% impurity (w/w). In some embodiments, the ester derivative contains less than 0.3% impurity (w/w). In some embodiments, the ester derivative contains less than 0.2% impurity (w/w). In some embodiments, the ester derivative contains less than 0.1% impurity (w/w).

In some embodiments, the ester derivative contains less than 250 ppm of any metals, less than 240 ppm of any metals, less than 230 ppm of any metals, less than 220 ppm of any metals, less than 210 ppm of any metals, less than 200 ppm of any metals, less than 190 ppm of any metals, less than 180 ppm of any metals, less than 170 ppm of any metals, less than 160 ppm of any metals, less than 150 ppm of any metals, less than 140 ppm of any metals, less than 130 ppm of any metals, less than 120 ppm of any metals, less than 110 ppm of any metals, less than 100 ppm of any metals, less than 90 ppm of any metals, less than 80 ppm of any metals, less than 70 ppm of any metals, less than 60 ppm of any metals, less than 50 ppm of any metals, less than 40 ppm of any metals, less than 30 ppm of any metals, less than 20 ppm of any metals, less than 10 ppm of any metals, less than 5 ppm of any metals, less than 4 ppm of any metals, less than 3 ppm of any metals, less than 2 ppm of any metals, less than 1 ppm of any metals, less than 0.9 ppm of any metals, less than 0.8 ppm of any metals, less than 0.7 ppm of any metals, less than 0.6 ppm of any metals, less than 0.5 ppm of any metals, less than 0.4 ppm of any metals, less than 0.3 ppm of any metals, less than 0.2 ppm of any metals, less than 0.1 ppm of any metals, less than 0.09 ppm of any metals, less than 0.08 ppm of any metals, less than 0.07 ppm of any metals, less than 0.06 ppm of any metals, less than 0.05 ppm of any metals, less than 0.04 ppm of any metals, less than 0.03 ppm of any metals, less than 0.02 ppm of any metals, or less than 0.01 ppm of any metals.

### GLYCOL

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

In some embodiments, the process further comprises the formation of a glycol. In some embodiments, the glycol is mono ethylene glycol (MEG) (or ethylene glycol). In some embodiments, the glycol is propylene glycol. In some embodiments, the glycol is butylene glycol.

In some embodiments, the glycol is obtained in between 80 and 99 mol% yield. In some embodiments, the glycol is obtained in between 85 and 99 mol% yield. In some embodiments, the glycol is obtained in between 90 and 99 mol% yield. In some embodiments, the glycol is obtained in at least 80 mol% yield. In some embodiments, the glycol is obtained in at least 85 mol% yield.

In some embodiments, the glycol contains less than 10% impurity (w/w). In some embodiments, the glycol contains less than 9% impurity (w/w). In some embodiments, the glycol contains less than about 8% impurity (w/w). In some embodiments, the glycol contains less than 7% impurity (w/w). In some embodiments, the glycol contains less than 6% impurity (w/w). In some embodiments, the glycol contains less than 5% impurity (w/w). In some embodiments, the glycol contains less than 4% impurity (w/w). In some embodiments, the glycol contains less than 3% impurity (w/w). In some embodiments, the glycol contains less than 2% impurity (w/w). In some embodiments, the glycol contains less than 1% impurity (w/w). In some embodiments, the glycol contains less than 0.5% impurity (w/w). In some embodiments, the glycol contains less than 0.4% impurity (w/w). In some embodiments, the glycol contains less than 0.3% impurity (w/w). In some embodiments, the glycol contains less than 0.2% impurity (w/w). In some embodiments, the glycol contains less than 0.1% impurity (w/w).

In some embodiments, the glycol contains less than 250 ppm of any metals, less than 240 ppm of any metals, less than 230 ppm of any metals, less than 220 ppm of any metals, less than 210 ppm of any metals, less than 200 ppm of any metals, less than 190 ppm of any metals, less than 180 ppm of any metals, less than 170 ppm of any metals, less than 160 ppm of any metals, less than 150 ppm of any metals, less than 140 ppm of any metals, less than 130 ppm of any metals, less than 120 ppm of any metals, less than 110 ppm of any metals, less than 100 ppm of any metals, less than 90 ppm of any metals, less than 80 ppm of any metals, less than 70 ppm of any metals, less than 60 ppm of any metals, less than 50 ppm of any metals, less than 40 ppm of any metals, less than 30 ppm of any metals, less than 20 ppm of any metals, less than 10 ppm of any metals, less than 5 ppm of any metals, less than 4 ppm of any metals, less than 3 ppm of any metals, less than 2 ppm of any metals, less than 1 ppm of any metals, less than 0.9 ppm of any metals, less than 0.8 ppm of any metals, less than 0.7 ppm of any metals, less than 0.6 ppm of any metals, less than 0.5 ppm of any metals, less than 0.4 ppm of any metals, less than 0.3 ppm of any metals, less than 0.2 ppm of any metals, less than .1 ppm of any metals, less than 0.09 ppm of any metals, less than 0.08 ppm of any metals, less than 0.07 ppm of any metals, less than 0.06 ppm of any metals, less than 0.05 ppm of any metals, less than 0.04 ppm of any metals, less than 0.03 ppm of any metals, less than 0.02 ppm of any metals, or less than 0.01 ppm of any metals.

### ALCOHOLIC SOLVENT

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

The process described herein comprises an alcoholic solvent. In some embodiments, the alcoholic solvent is a linear alcohol, branched alcohol, cyclic alcohol, or any combinations thereof. In some embodiments, the alcoholic solvent is selected from methanol, ethanol, n-propanol, isopropanol, t-butanol, ethylene glycol, glycerol, cyclohexane-1,4-diyldimethanol, phenol, benzyl alcohol, and any combinations thereof.

In some embodiments, the alcoholic solvent is a linear C₁-C₄ alcohol. In some embodiments, the alcoholic solvent is methanol, ethanol, propanol, butanol, or a combination thereof. In some embodiments, the alcoholic solvent is methanol, ethanol, propanol, or a combination thereof. In some embodiments, the alcoholic solvent is methanol. In some embodiments, the alcohol is ethanol. In some embodiments, the alcoholic solvent is a branched C₃-C₄ alcohol. In some embodiments, the alcoholic solvent is t-butanol, s-butanol, i-butanol, i-propanol, or any combinations thereof. In some embodiments, the alcoholic solvent is a cyclic C₃-C₈ alcohol. In some embodiments, the alcoholic solvent is cyclopropanol, cyclobutanol, cyclopentanol, cyclohexanol, cycloheptanol, cyclohexane-1,4-diyldimethanol, or any combinations thereof. In some embodiments, the alcoholic solvent is cyclohexane-1,4-diyldimethanol.

In some embodiments, the alcoholic solvent is a polyol. In some embodiments, the alcoholic solvent is selected from ethylene glycol, glycerol, and any combinations thereof.

In some embodiments, the alcoholic solvent is selected from phenol, benzyl alcohol, and any combinations thereof.

### SOLVENT FOR SWELLING THE POLYESTER

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

The process described herein comprises pre-treating the polyester with a solvent for swelling the polyester prior to the addition of the alcoholic solvent and alkoxide.The pre-treatment with the solvent for swelling the polyester is done for between 5 mins and 60 mins prior to the addition of the alcoholic solvent and alkoxide. In some embodiments, the pre-treatment with the solvent for swelling the polyester is done for between 5 mins and 40 mins prior to the addition of the alcoholic solvent and alkoxide. In some embodiments, the pre-treatment with the solvent for swelling the polyester is done for between 5 mins and 20 mins prior to the addition of the alcoholic solvent and alkoxide. In some embodiments, the pre-treatment with the solvent for swelling the polyester is done for between 5 mins and 10 mins prior to the addition of the alcoholic solvent and alkoxide. In some embodiments, the pre-treatment with the solvent for swelling the polyester is done for 5 mins. In some embodiments, the pre-treatment with the solvent for swelling the polyester is done for 10 mins. In some embodiments, the pre-treatment with the solvent for swelling the polyester is done for 15 mins. In some embodiments, the pre-treatment with the solvent for swelling the polyester is done for 20 mins. In some embodiments, the pre-treatment with the solvent for swelling the polyester is done for 25 mins. In some embodiments, the pre-treatment with the solvent for swelling the polyester is done for 30 mins. In some embodiments, the pre-treatment with the solvent for swelling the polyester is done for 35 mins. In some embodiments, the pre-treatment with the solvent for swelling the polyester is done for 40 mins. In some embodiments, the pre-treatment with the solvent for swelling the polyester is done for 45 mins. In some embodiments, the pre-treatment with the solvent for swelling the polyester is done for 50 mins. In some embodiments, the pre-treatment with the solvent for swelling the polyester is done for 55 mins. In some embodiments, the pre-treatment with the solvent for swelling the polyester is done for 60 mins.

In some embodiments, the solvent for swelling the polyester is selected from a non polar solvent, a polar aprotic solvent, a polar protic solvent, and any combinations thereof.

In some embodiments, the solvent for swelling the polyester is selected from dimethyl sulfoxide (DMSO), *N,N-*dimethylformamide (DMF), acetone, a halogenated solvent, n-hexane, nitrobenzene, methanol, benzyl alcohol, benzaldehyde, and any combinations thereof. In some embodiments, the solvent for swelling the polyester is selected from DMSO, a halogenated solvent, and any combinations thereof. In some embodiments, the solvent for swelling the polyester is DMSO. In some embodiments, the solvent for swelling the polyester is a halogenated solvent. In some embodiments, the solvent for swelling the polyester is a chlorinated solvent. In some embodiments, the solvent for swelling the polyester is dichloromethane, dichloroethane, tetrachloroethane, chloroform, tetrachloromethane, trichloroethane, or any combinations thereof. In some embodiments, the solvent for swelling the polyester is dichloromethane. In some embodiments, the solvent for swelling the polyester is methanol.

In some embodiments, the ratio of solvent for swelling the polyester to alcoholic solvent is between 0.1:1 and 2:1 (w:w). In some embodiments, the ratio of solvent for swelling the polyester to alcoholic solvent is between 1:1 and 2:1 (w:w). In some embodiments, the ratio of solvent for swelling the polyester to alcoholic solvent is between 0.1:1 and 2:1 (w:w), or between 0.2:1 and 2:1 (w:w), or between 0.3:1 and 2:1 (w:w), or between 0.4:1 and 2:1 (w:w), or between 0.5:1 and 2:1 (w:w), or between 0.6:1 and 2:1 (w:w), or between 0.7:1 and 2:1 (w:w), or between 0.8:1 and 2:1 (w:w), or between 0.9:1 and 2:1 (w:w), or between 1:1 and 2:1 (w:w), or between 1:2 and 2:1 (w:w), or between 1:3 and 2:1 (w:w), or between 1:4 and 2:1 (w:w), or between 1:4 and 2:1 (w:w), or between 1:6 and 2:1 (w:w), or between 1:7 and 2:1 (w:w), or between 1:8 and 2:1 (w:w), or between 1:9 and 2:1 (w:w), or between 0.5:1 and 1.5:1 (w:w), or between 0.8:1 and 1.2:1 (w:w), or between 0.5:1 and 1:1 (w:w), or between 1:1 and 1.5:1 (w:w). In some embodiments, the ratio of solvent for swelling the polyester to alcoholic solvent is between 0.5:1 and 1:1 (w:w).

### ALKOXIDES

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

The process described herein comprises adding a sub-stoichiometric amount of an alkoxide. In some embodiments, the process described herein comprises adding a catalytic amount of an alkoxide.

"Sub-stoichiometric amount", as used herein, is used to indicate that the amount of material used is less than a stoichiometric amount. The term is used herein interchangeably with "catalytic amount." In some embodiments, a sub-stoichiometric amount is less than or equal to 95% of a stoichiometric amount. In some embodiments, a sub-stoichiometric amount is less than or equal to 90% of a stoichiometric amount. In some embodiments, a sub-stoichiometric amount is less than or equal to 85% of a stoichiometric amount. In some embodiments, a sub-stoichiometric amount is less than or equal to 80% of a stoichiometric amount. In some embodiments, a sub-stoichiometric amount is less than or equal to 75% of a stoichiometric amount. In some embodiments, a sub-stoichiometric amount is less than or equal to 70% of a stoichiometric amount. In some embodiments, a sub-stoichiometric amount is less than or equal to 65% of a stoichiometric amount. In some embodiments, a sub-stoichiometric amount is less than or equal to 60% of a stoichiometric amount. In some embodiments, a sub-stoichiometric amount is less than or equal to 55% of a stoichiometric amount. In some embodiments, a sub-stoichiometric amount is less than or equal to 50% of a stoichiometric amount. In some embodiments, a sub-stoichiometric amount is less than or equal to 45% of a stoichiometric amount. In some embodiments, a sub-stoichiometric amount is less than or equal to 40% of a stoichiometric amount. In some embodiments, a sub-stoichiometric amount is less than or equal to 35% of a stoichiometric amount. In some embodiments, a sub-stoichiometric amount is less than or equal to 30% of a stoichiometric amount. In some embodiments, a sub-stoichiometric amount is less than or equal to 25% of a stoichiometric amount. In some embodiments, a sub-stoichiometric amount is less than or equal to 20% of a stoichiometric amount. In some embodiments, a sub-stoichiometric amount is less than or equal to 15% of a stoichiometric amount. In some embodiments, a sub-stoichiometric amount is less than or equal to 10% of a stoichiometric amount.

"Stoichiometric amount", as used herein, is used to indicate that the amount of material used is equivalent to the number of ester linkages present in the polyester.

In some embodiments, the alkoxide, which comprises an alkoxide anion and a cation is selected from an alkali metal alkoxide, an alkaline earth metal alkoxide, a metal alkoxide, an ammonium alkoxide, and any combinations thereof. In some embodiments, the alkoxide anion is a C₁-C₄ alkoxide anion. In some embodiments, the alkoxide anion is selected from methoxide, ethoxide, n-propoxide, n-butoxide, t-butoxide, sec-butoxide, iso-butoxide, iso-propoxide, and a combination thereof. In some embodiments, the alkoxide anion is methoxide, ethoxide, or any combinations thereof. In some embodiments, the alkoxide anion is methoxide. In some embodiments, the cation is lithium, sodium, potassium, magnesium, calcium, strontium, barium, zinc, aluminum, or ammonium. In some embodiments, the alkoxide is sodium methoxide, potassium ethoxide, or aluminum tri-n-propoxide. In some embodiments, the ammonium alkoxide is a tetraalkylammonium alkoxide. In some embodiments, the ammonium alkoxide is a tetrabutylammonium alkoxide. In some embodiments, the alkoxide is sodium methoxide, potassium ethoxide, aluminum tri-n-propoxide, or tetrabutylammonium methoxide.

In some embodiments, the alkoxide is generated in-situ by addition of an alkali metal, an alkaline earth metal, or a metal to the alcoholic solvent.

The ratio of polyester to alkoxide is between 20:1 and 125:1 (w:w). In some embodiments, the ratio of polyester to alkoxide is between 20:1 and 25:1 (w:w), or between 20:1 and 50:1 (w:w), or between 30:1 and 60:1 (w:w), or between 40:1 and 70:1 (w:w), or between 50:1 and 80:1 (w:w), or between 60:1 and 90:1 (w:w), or between 20:1 and 125:1 (w:w), or between 40:1 and 125:1 (w:w), or between 60:1 and 125:1 (w:w), or between 80:1 and 125:1 (w:w), or between 100:1 and 125:1 (w:w). In some embodiments, the ratio of polyester to alkoxide is between 20:1 and 25:1 (w:w).

### REACTION TIME

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

In some embodiments, the admixing of the polyester with the alkoxide and the alcoholic solvent is performed for a sufficient time. In some embodiments, the admixing of the polyester with the alkoxide and the alcoholic solvent is performed until an 70% yield of desired ester is achieved. In some embodiments, the admixing of the polyester with the alkoxide and the alcoholic solvent is performed until an 75% yield of desired ester is achieved. In some embodiments, the admixing of the polyester with the alkoxide and the alcoholic solvent is performed until an 80% yield of desired ester is achieved. In some embodiments, the admixing of the polyester with the alkoxide and the alcoholic solvent is performed until an 85% yield of desired ester is achieved. In some embodiments, the admixing of the polyester with the alkoxide and the alcoholic solvent is performed until an 90% yield of desired ester is achieved. In some embodiments, the admixing of the polyester with the alkoxide and the alcoholic solvent is performed until an 95% yield of desired ester is achieved.

In some embodiments, the admixing of the polyester with the alkoxide and the alcoholic solvent is performed for between 10 mins and 5 hrs, or between 10 mins and 4 hrs, or between 10 mins and 3 hrs, or between 10 mins and 2 hrs, or between 20 mins and 2 hrs, or between 30 mins and 2hrs, or between 40 mins and 2 hrs, or between 30 mins and 1 hr, or between 30 mins and 1.5 hrs. In some embodiments, the admixing of the polyester with the alkoxide and the alcoholic solvent is performed for 10 mins, or 15 mins, or 20 mins, or 25 mins, or 30 mins, or 35 mins, or 40 mins, or 45 mins, or 50 mins, or 60 mins, or 70 mins, or 80 mins, or 90 mins, or 100 mins, or 110 mins, or 120 mins, or 130 mins, or 140 mins, or 150 mins, or 160 mins, or 170 min, or 180 mins. In some embodiments, the admixing of the polyester with the alkoxide and the alcoholic solvent is performed for 1 hr, or 2 hrs, or 3 hrs, 4 hrs, or 5 hrs.

### TEMPERATURE

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

The process disclosed herein may be conducted at ambient temperature. Ambient temperature may be 25±5 °C.

In some embodiments, the process disclosed herein is conducted without external heat.

In some embodiments, the process is exothermic and the temperature of the reaction mixture rises to at least 30 °C, at least 35 °C, at least 40 °C, at least 45 °C, at least 50 °C, at least 55 °C, or at least 60 °C. In some embodiments, no external heat sources are used to increase the temperature of the reaction mixture.

In some embodiments, the process disclosed herein is conducted with external heat. In some embodiments, the process disclosed herein is conducted with external heat at between 25 °C and 100 °C, or between 25 °C and 85 °C, or between 25 °C and 80 °C, or between 25 °C and 60 °C, or between 40 °C and 60 °C, or between 40 °C and 50 °C, or between 30 °C and 50 °C. In some embodiments, the process disclosed herein is conducted at 26 °C, 27 °C, 28 °C, 29 °C, 30 °C, 31 °C, 32 °C, 33 °C, 34 °C, 35 °C, 36 °C, 37 °C, 38 °C, 39 °C, 40°C, 41 °C, 42 °C, 43 °C, 44 °C, 45 °C, 46 °C, 47°C, 48 °C, 49 °C, 50 °C, 51 °C, 52 °C, 53 °C, 54 °C, 55 °C, 56 °C, 57 °C, 58 °C, 59 °C, 60 °C, 61 °C, 62 °C, 63 °C, 64 °C, 65 °C, 66 °C, 67 °C, 68 °C, 69 °C, 70 °C, 71 °C, 72 °C, 73 °C, 74 °C, 75 °C, 76 °C, 77 °C, 78 °C, 79 °C, 80 °C, 81 °C, 82 °C, 83 °C, 84 °C, 85 °C, 86 °C, 87 °C, 88 °C, 89 °C, 90 °C, 91 °C, 92 °C, 93 °C, 94 °C, 95 °C, 96 °C, 97 °C, 98 °C, 99 °C, or 100 °C.

### PRESSURE

In some embodiments, the process disclosed herein is conducted at atmospheric pressure. In some embodiments, the process disclosed herein is conducted at elevated pressures. In some embodiments, the process disclosed herein is conducted at a pressure between atmospheric pressure and 1517 kPa (220 psi), or between atmospheric pressure and 1379 kPa (200 psi), or between atmospheric pressure and 1034 kPa (150 psi), or between atmospheric pressure and 689 kPa (100 psi), or between atmospheric pressure and 344 kPa (50 psi), or between 138 kPa (20 psi) and 1034 kPa (150 psi), or between 344 kPa (50 psi) and 689 kPa (100 psi). In some embodiments, the process disclosed herein is conducted at 97 kPa (14 psi), 103 kPa (15 psi), 110 kPa (16 psi), 117 kPa (17 psi), 124 kPa (18 psi), 131 kPa (19 psi), 138 kPa (20 psi), 207 kPa (30 psi), 276 kPa (40 psi), 344 kPa (50 psi), 414 kPa (60 psi), 483 kPa (70 psi), 552 kPa (80 psi), 621 kPa (90 psi), 689 kPa (100 psi), 758 kPa (110 psi), 827 kPa (120 psi), 896 kPa (130 psi), 965 kPa (140 psi), 1034 kPa (150 psi), 1103 kPa (160 psi), 1172 kPa (170 psi), about 1241 kPa (180 psi), 1310 kPa (190 psi), 1379 kPa (200 psi), 1448 kPa (210 psi), or 1517 kPa (220 psi).

### AGITATION

Described herein is a process for the transformation of a polyester into an ester derivative; the process comprising pre-treating the polyester with a solvent for swelling the polyester, and admixing the polyester with a mixture comprising:
(a) an alcoholic solvent; and
(b) a sub-stoichiometric amount of an alkoxide.

In some embodiments, the process disclosed herein is conducted without agitation. In some embodiments, the process disclosed herein is conducted with increased agitation. In some embodiments, a stirred batch reactor is used to provide agitation. In some embodiments, a continuous reactor is used to provide agitation.

### CERTAIN TERMINOLOGY

The section headings used herein are for organizational purposes only and are not to be construed as limiting the subject matter described.

Unless defined otherwise, all technical and scientific terms used herein have the same meaning as is commonly understood to which the claimed subject matter belongs. In the event that there are a plurality of definitions for terms herein, those in this section prevail.

It is to be understood that the general description and the detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

Unless the context requires otherwise, throughout the specification and claims which follow, the word "comprise" and variations thereof, such as, "comprises" and "comprising" are to be construed in an open, inclusive sense, that is, as "including, but not limited to." Further, headings provided herein are for convenience only and do not interpret the scope or meaning of the claimed invention.

As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

As used herein, the term "about" or "approximately" means within 10%, preferably within 10%, and more preferably within 5% of a given value or range.

As used herein, ambient temperature is a colloquial expression for the typical or preferred indoor (climate-controlled) temperature to which people are generally accustomed. It represents the small range of temperatures at which the air feels neither hot nor cold, approximately 21 °C. In some embodiments, ambient temperature is 25±5 °C. In some embodiments, ambient temperature is 18 °C. In some embodiments, ambient temperature is 19 °C. In some embodiments, ambient temperature is 20 °C. In some embodiments, ambient temperature is 21 °C. In some embodiments, ambient temperature is 22 °C. In some embodiments, ambient temperature is 23 °C. In some embodiments, ambient temperature is 24 °C. In some embodiments, ambient temperature is 25 °C. In some embodiments, ambient temperature is 26 °C. In some embodiments, ambient temperature is 27 °C. In some embodiments, ambient temperature is 28 °C. In some embodiments, ambient temperature is 29 °C. In some embodiments, ambient temperature is 30 °C.

As used in this specification and the appended claims, depolymerization, refer to a way of breaking down a polymer to its starting material. It is essentially the opposite of polymerization. In some embodiments, the depolymerization is achieved by glycolysis, methanolysis or hydrolysis, categorized by the depolymerization reactant used, such as glycol, methanol or water, respectively.

Definition of standard chemistry terms may be found in reference works, including but not limited to, Carey and Sundberg "Advanced Organic Chemistry 4th Ed." Vols. A (2000) and B (2001), Plenum Press, New York.

The terms below, as used herein, have the following meanings, unless indicated otherwise:
"Alkyl" refers to a straight or branched hydrocarbon chain radical which is attached to the rest of the molecule by a single bond. A linear alkyl comprising up to 4 carbon atoms is referred to as a linear C₁-C₄ alkyl, likewise, for example, a linear alkyl comprising up to 3 carbon atoms is a linear C₁-C₃ alkyl. Linear alkyl groups include linear C₁-C₄ alkyl, linear C₁-C₃ alkyl, linear C₁-C₂ alkyl, linear C₂-C₃ alkyl and linear C₂-C₄ alkyl. Representative alkyl groups include, methyl, ethyl, propyl, and butyl. A branched alkyl comprising between 3 and 4 carbon atoms is referred to as a branched C₃-C₄ alkyl. Representative branched alkyl groups include, but are not limited to t-butyl, sec-butyl, isobutyl, and isopropyl. Unless stated otherwise specifically in the specification, an alkyl is optionally substituted with oxo, halogen, -CN, -CF₃, -OH, - OMe, -NH₂, or -NO₂. In some embodiments, an alkyl is optionally substituted with oxo, halogen, -CN, -CF₃, -OH, or -OMe. In some embodiments, the alkyl is optionally substituted with halogen.

"Halo" or "halogen" refers to bromo, chloro, fluoro or iodo. In some embodiments, halogen is fluoro or chloro. In some embodiments, halogen is fluoro.

"Haloalkyl" refers to an alkyl radical, as defined above, that is substituted by one or more halo atoms, as defined above, e.g., trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl, and the like.

"Hydroxyalkyl" refers to an alkyl radical, as defined above, that is substituted by one or more -OH groups, e.g., hydroxymethyl, hydroxyethyl, hydroxypropyl, propanediol, and the like.

"Cycloalkyl" refers to a stable, partially or fully saturated, monocyclic or polycyclic carbocyclic ring, which may include fused or bridged ring systems. Representative cycloalkyls include, but are not limited to, cycloalkyls having from three to fifteen carbon atoms (C₃-C₁₅ cycloalkyl), from three to ten carbon atoms (C₃-C₁₀ cycloalkyl), from three to eight carbon atoms (C₃-C₈ cycloalkyl), from three to six carbon atoms (C₃-C₆ cycloalkyl), from three to five carbon atoms (C₃-C₅ cycloalkyl), or three to four carbon atoms (C₃-C₄ cycloalkyl). In some embodiments, the cycloalkyl is a 3- to 6-membered cycloalkyl. In some embodiments, the cycloalkyl is a 5- to 6-membered cycloalkyl. Monocyclic cycloalkyls include, for example, cyclopropyl, cyclobutyl, cyclopentyl, cyclohexyl, cycloheptyl, and cyclooctyl. Polycyclic cycloalkyls or carbocycles include, for example, adamantyl, norbornyl, decalinyl, bicyclo[3.3.0]octane, bicyclo[4.3.0]nonane, cis-decalin, trans-decalin, bicyclo[2.1.1]hexane, bicyclo[2.2.1]heptane, bicyclo[2.2.2]octane, bicyclo[3.2.2]nonane, and bicyclo[3.3.2]decane, and 7,7-dimethyl-bicyclo[2.2.1]heptanyl. Partially saturated cycloalkyls include, for example cyclopentenyl, cyclohexenyl, cycloheptenyl, and cyclooctenyl. Unless stated otherwise specifically in the specification, a cycloalkyl is optionally substituted with oxo, halogen, methyl, ethyl, -CN, -CF₃, -OH, -OMe, -NH₂, or -NO₂. In some embodiments, a cycloalkyl is optionally substituted with oxo, halogen, methyl, ethyl, - CN, -CF₃, -OH, or -OMe. In some embodiments, the cycloalkyl is optionally substituted with halogen.

"Aryl" refers to a radical derived from a hydrocarbon ring system comprising hydrogen, 6 to 30 carbon atoms and at least one aromatic ring. The aryl radical may be a monocyclic, bicyclic, tricyclic or tetracyclic ring system, which may include fused or bridged ring systems. In some embodiments, the aryl is a 6- to 10-membered aryl. In some embodiments, the aryl is a 6-membered aryl. Aryl radicals include, but are not limited to, aryl radicals derived from the hydrocarbon ring systems of anthrylene, naphthylene, phenanthrylene, anthracene, azulene, benzene, chrysene, fluoranthene, fluorene, as-indacene, s-indacene, indane, indene, naphthalene, phenalene, phenanthrene, pleiadene, pyrene, and triphenylene. In some embodiments, the aryl is phenyl. Unless stated otherwise specifically in the specification, an aryl may be optionally substituted, for example, with halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, an aryl is optionally substituted with halogen, methyl, ethyl, -CN, -CF₃, -OH, -OMe, -NH₂, or -NO₂. In some embodiments, an aryl is optionally substituted with halogen, methyl, ethyl, -CN, -CF₃, -OH, or -OMe. In some embodiments, the aryl is optionally substituted with halogen.

As used herein, the term "mol" when referring to PET is the molar amount and is calculated using the molecular weight of the "PET" unit which is 192.17 g/mol.

### EXAMPLES

The following examples are intended to illustrate but not limit the disclosed embodiments.

### Example 1:

Polyethylene terephthalate (1000g) was introduced in a reactor. Dichloromethane (500g) was added and the mixture was stirred at room temperature and at atmospheric pressure for about 40 mins. Sodium methoxide and methanol were then added to the reaction mixture was stirred and heated for 120 mins (see table below for amounts, time, and temperature details).

The reaction mixture was then filtered and the filter cake was washed with methanol. The filter cake was then melted and filtered at 140 °C to remove any unreacted materials. The filtered dimethyl terephthalate was then distilled under vacuum at 200 °C. The liquid recovered from the filtration was distilled to recover the solvents and the mono ethylene glycol.

| **Ex** | **Weight of methanol (g)** | **Weight of sodium methoxide (g)** | **Reaction time (min)** | **Reaction temperature (°C)** | **Yield (%)** |
|---|---|---|---|---|---|
| **1A** | 667 | 32 | 120 | 55 | 90 |
| **1B** | 600 | 54 | 120 | 50 | 90 |
| **1C** | 580 | 50 | 120 | 60 | 90 |

### Example 2:

Polyethylene terephthalate (1000g) was introduced in a reactor. DMSO (500g) was added and the mixture was stirred at room temperature and at atmospheric pressure for about 40 mins. Sodium methoxide (45g) and methanol (550g) were then added to the reaction mixture was stirred and heated at 55 °C for 120 mins.

The reaction mixture was then filtered and the filter cake was washed with methanol.

The filter cake was then melted and filtered at 140 °C to remove any unreacted materials. The filtered dimethyl terephthalate was then distilled under vacuum at 200 °C. The liquid recovered from the filtration was distilled to recover the solvents and the mono ethylene glycol.

Dimethyl terephthalate was obtained in 89% yield.

### Example 3:

PET (10000g) was treated with methanol (6000g) at 60°C for an hour. The methanol was then drained. After draining, the reactor was heated up from 60°C to 70°C and the sodium methoxide (at 25%wt in methanol) was added. Thirty minutes after the sodium methoxide addition, the balance of methanol was added. The reaction was stopped 3 hrs after the addition of the sodium methoxide. The reactional mixture was cooled at room temperature then filtered to separate MEG, sodium methoxide, and residual methanol from DMT and unreacted material. The filtration worked for trials 3 to 5 while trials 1 and 2 were unfilterable even with using different solid liquid separation methods (centrifugation, press filter, vacuum filter, or pressure filter). Since filtration was not possible, distillation of methanol followed with distillation of MEG and then distillation of DMT from the reactional mixture was tried in a rotary evaporator. After recovery of the methanol the reactional mixture turned into a paste from where distillation of MEG and DMT was not possible due to side reactions with contaminants.

For trials 3 to 5, after solid-liquid separation. The residual methanol and MEG were recovered from the liquid phase into two different fractions using a rotary evaporator. DMT was successfully recovered from the solid phase using a thin film evaporator.

The table below shows the recovery of DMT and MEG with varying amounts of sodium methoxide.

| **Trial** | **NaOMe (g)** | **PET: NaOMe Ratio (w/w)** | **Recovery of Pure DMT¹** | **Recovery of Pure MEG²** |
|---|---|---|---|---|
| 1 | 2000 | 5:1 | No³ | No³ |
| 2 | 1000 | 10:1 | No³ | No³ |
| 3 | 500 | 20:1 | Yes⁴ | Yes⁴ |
| 4 | 300 | 33:1 | Yes⁴ | Yes⁴ |
| 5 | 200 | 50:1 | Yes⁴ | Yes⁴ |

| | | | | |
|---|---|---|---|---|
| ¹ dimethyl terephthalate ² mono ethylene glycol ³ The slurry was heated to try evaporating the DMT and the MEG but it wasn't possible due products generation mixed with all the contaminants. ⁴ Pure DMT was recovered from the cake that was exempt of MEG and catalyst. Pure MEG was recovered from the mother liquor and the washes that contained less than 1% DMT. | | | | |

## Claims

1. A process for the transformation of a polyester selected from the group consisting of polyethylene terephthalate and polyethylene glycol-co-1,4-cyclohexanedimethanol terephthalate) into a terephthalate of Formula (I): 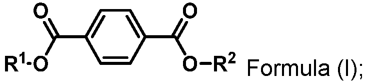
wherein R¹ and R² are independently selected from the group consisting of hydrogen, C₁-C₆ alkyl, C₁-C₆ haloalkyl, C₁-C₆ hydroxyalkyl, C₃-C₈ cycloalkyl, (C₁-C₆ alkyl)(C₃-C₈ cycloalkyl), aryl, and (C₁-C₆ alkyl)(aryl); provided that one of R¹ or R² is not hydrogen; wherein C₃-C₈ cycloalkyl is optionally substituted with oxo, halogen, methyl, ethyl, -CN, -CF₃, -OH, -OMe, -NH₂, or -NO₂;
wherein C₁-C₆ alkyl is optionally substituted with oxo, halogen, -CN, -CF₃, -OH, -OMe, -NH₂, or-NO₂; and
wherein aryl is optionally substituted with halogen, amino, nitrile, nitro, hydroxyl, alkyl, alkenyl, alkynyl, haloalkyl, alkoxy, aryl, cycloalkyl, heterocycloalkyl, heteroaryl, -CN, -CF₃, or -OMe,
wherein cycloalkyl in the definitions above refers to a partially or fully saturated, monocyclic or polycyclic carbocyclic ring;
the process comprising pre-treating the polyester with a solvent for swelling the polyester, and admixing the polyester with a mixture comprising:
(a) an alcoholic solvent; and
(b) a sub-stoichiometric amount of an alkoxide wherein the process is performed at a temperature between 25 °C and 100 °C;
wherein the ratio of the polyester to the alkoxide is between 20:1 and 125:1 (w:w); and
wherein the pre-treating the polyester with the solvent for swelling the polyester is performed for between 5 mins and 60 mins prior to admixing the polyester with the mixture comprising (a) and (b).

2. The process of claim 1, wherein R¹ or R² is methyl.

3. The process of claim 1 or 2, wherein the alcoholic solvent is methanol.

4. The process of any one of claims 1-3, wherein the solvent for swelling the polyester is a methanol.

5. The process of any one of claims 1-4, wherein the ratio of the solvent for swelling the polyester to the alcoholic solvent is between 0.1:1 and 2:1 (w:w).

6. The process of any one of claims 1-5, wherein the alkoxide anion is methoxide.

7. The process of any one of claims 1-6, wherein the ratio of the polyester to the alkoxide is between 20:1 and 50:1 (w:w).

8. The process of any one of claims 1-7, wherein the process is performed until at least 70% yield of the terephthalate of Formula (I) is achieved.

9. The process of any one of claims 1-8, wherein the process is performed until at least 80% yield of the terephthalate of Formula (I) is achieved.

10. The process of any one of claims 1-9, wherein the process is performed at atmospheric pressure.

11. The process of any one of claims 1-10, wherein the polyester is polyethylene terephthalate.

12. The process of any one of claims 1-11, wherein the process further comprises recovering monoethylene glycol (MEG).

13. The process of any one of claims 1-12, wherein the polyester is in the form of polymer flakes and is part of a feedstock.

14. The process of claim 13, wherein the average polymer flakes particle size is between 1 mm and 20 mm.

15. The process of any one of claims 1-14, wherein the process is performed at a pressure between atmospheric pressure and 1379 kPa.

## Patentansprüche

1. Verfahren zur Umwandlung eines Polyesters, der aus der aus Polyethylenterephthalat und Poly(ethylenglykol-co-1,4-cyclohexandimethanolterephthalat) bestehenden Gruppe ausgewählt ist, in ein Terephthalat der Formel (I): 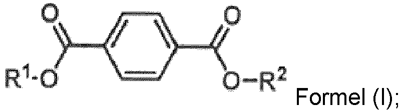
worin R¹ und R² jeweils unabhängig voneinander aus der aus Wasserstoff, C₁-C₆-Alkyl, C₁-C₆-Halogenalkyl, C₁-C₆-Hydroxyalkyl, C₃-C₈-Cycloalkyl, (C₁-C₆-Alkyl)(C₃-C₈-cycloalkyl), Aryl und (C₁-C₆-Alkyl)(aryl) bestehenden Gruppe ausgewählt sind; mit der Maßgabe, dass einer von R¹ und R² nicht Wasserstoff ist,
wobei das C₃-C₈-Cycloalkyl gegebenenfalls mit Oxo, Halogen, Methyl, Ethyl, -CN-, -CF₃, -OH, -OMe, -NH₂ oder -NO₂ substituiert ist,
wobei das C₁-C₆-Alkyl gegebenenfalls mit Oxo, Halogen, -CN, -CF₃, -OH, -OMe, -NH₂ oder -NO₂ substituiert ist, und
wobei das Aryl gegebenenfalls mit Halogen, Amino, Nitril, Nitro, Hydroxyl, Alkyl, Alkenyl, Alkinyl, Halogenalkyl, Alkoxy, Aryl, Cycloalkyl, Heterocycloalkyl, Heteroaryl, -CN, -CF₃ oder -OMe substituiert ist;
wobei sich das Cycloalkyl in den vorstehenden Definitionen auf einen teilweise oder vollständig gesättigten, monozyklischen oder polyzyklischen Carbozyklus bezieht;
wobei das Verfahren das Vorbehandeln des Polyesters mit einem Lösungsmittel zum Quellen des Polyesters und das Vermischen des Polyesters mit einem Gemisch umfasst, das Folgendes umfasst:
(a) ein alkoholisches Lösungsmittel und
(b) eine substöchiometrische Menge eines Alkoxids;
wobei das Verfahren bei einer Temperatur zwischen 25 °C und 100 °C ausgeführt wird; wobei das Verhältnis zwischen dem Polyester und dem Alkoxid zwischen 20:1 und 125:1 (Gew.:Gew.) beträgt; und
wobei das Vorbehandeln des Polyesters mit dem Lösungsmittel zum Quellen des Polyesters zwischen 5 min und 60 min lang durchgeführt wird, bevor der Polyester mit dem (a) und (b) umfassenden Gemisch vermischt wird.

2. Verfahren nach Anspruch 1, wobei R¹ oder R² Methyl ist.

3. Verfahren nach Anspruch 1 oder 2, wobei das alkoholische Lösungsmittel Methanol ist.

4. Verfahren nach einem der Ansprüche 1 bis 3, wobei das Lösungsmittel zum Quellen des Polyesters Methanol ist.

5. Verfahren nach einem der Ansprüche 1 bis 4, wobei das Verhältnis zwischen dem Lösungsmittel zum Quellen des Polyesters und dem alkoholischen Lösungsmittel zwischen 0,1:1 und 2:1 (Gew.:Gew.) beträgt.

6. Verfahren nach einem der Ansprüche 1 bis 5, wobei das Alkoxidanion Methoxid ist.

7. Verfahren nach einem der Ansprüche 1 bis 6, wobei das Verhältnis zwischen dem Polyester und dem Alkoxid zwischen 20:1 und 50:1 (Gew.:Gew.) beträgt.

8. Verfahren nach einem der Ansprüche 1 bis 7, wobei das Verfahren ausgeführt wird, bis zumindest 70 % Ausbeute des Terephthalats der Formel (I) erhalten wurden.

9. Verfahren nach einem der Ansprüche 1 bis 8, wobei das Verfahren ausgeführt wird, bis zumindest 80 % Ausbeute des Terephthalats der Formel (I) erhalten wurden.

10. Verfahren nach einem der Ansprüche 1 bis 9, wobei das Verfahren bei Atmosphärendruck ausgeführt wird.

11. Verfahren nach einem der Ansprüche 1 bis 10, wobei der Polyester Polyethylenterephthalat ist.

12. Verfahren nach einem der Ansprüche 1 bis 11, wobei das Verfahren weiters das Gewinnen von Monoethylenglykol (MEG) umfasst.

13. Verfahren nach einem der Ansprüche 1 bis 12, wobei der Polyester in Form von Polymerflocken vorliegt und Teil eines Beschickungsmaterials ist.

14. Verfahren nach Anspruch 13, wobei die mittlere Partikelgröße der Polymerflocken zwischen 1 mm und 20 mm beträgt.

15. Verfahren nach einem der Ansprüche 1 bis 14, wobei das Verfahren bei einem Druck zwischen Atmosphärendruck und 1379 kPa ausgeführt wird.

## Revendications

1. Procédé de transformation d'un polyester choisi dans le groupe constitué de polyéthylène téréphtalate et de poly(éthylène glycol-*co*-1,4-cyclohexanediméthanol téréphtalate) en un téréphtalate de formule (I) : 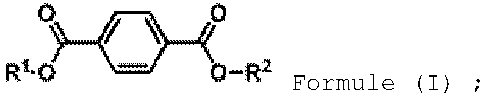
dans lequel R¹ et R² sont choisis indépendamment dans le groupe constitué de l'hydrogène, d'un alkyle en C₁-C₆, d'un haloalkyle en C₁-C₆, d'un hydroxyalkyle en C₁-C₆, d'un cycloalkyle en C₃-C₈, d'un (alkyle en C₁-C₆) (cycloalkyle en C₃-C₈), d'un aryle, et d'un (alkyle en C₁-C₆) (aryle) ;
à condition que l'un de R¹ ou R² ne soit pas de l'hydrogène ;
dans lequel un cycloalkyle en C₃-C₈ est facultativement substitué par un oxo, un halogène, un méthyle, un éthyle, - CN, -CF₃, -OH, -OMe, -NH₂ ou -NO₂;
dans lequel un alkyle en C₁-C₆ est facultativement substitué par un oxo, un halogène, -CN, -CF₃, -OH, -OMe, -NH₂ ou -NO₂, et
dans lequel l'aryle est facultativement substitué par un halogène, un amino, un nitrile, un nitro, un hydroxyle, un alkyle, un alcényle, un alcynyle, un haloalkyle, un alcoxy, un aryle, un cycloalkyle, un hétérocycloalkyle, un hétéroaryle, -CN, -CF₃ ou -OMe,
dans lequel le cycloalkyle, dans les définitions ci-dessus, fait référence à un cycle carbocyclique monocyclique ou polycyclique partiellement ou totalement saturé ;
le procédé comprenant un prétraitement du polyester avec un solvant pour faire gonfler le polyester, et un mélange du polyester avec un mélange comprenant :
(a) un solvant alcoolique ; et
(b) une quantité sous-stœchiométrique d'un alcoxyde
dans lequel le procédé est effectué à une température comprise entre 25°C et 100°C ; dans lequel le rapport du polyester par rapport à l'alcoxyde est compris entre 20:1 et 125:1 (p:p) ; et
dans lequel le prétraitement du polyester avec le solvant pour faire gonfler le polyester est effectué pendant 5 minutes à 60 minutes avant de mélanger le polyester avec le mélange comprenant (a) et (b).

2. Procédé selon la revendication 1, dans lequel R¹ ou R² est un méthyle.

3. Procédé selon la revendication 1 ou 2, dans lequel le solvant alcoolique est le méthanol.

4. Procédé selon l'une quelconque des revendications 1 à 3, dans lequel le solvant pour faire gonfler le polyester est un méthanol.

5. Procédé selon l'une quelconque des revendications 1 à 4, dans lequel le rapport du solvant pour faire gonfler le polyester au solvant alcoolique est compris entre 0,1:1 et 2:1 (p:p).

6. Procédé selon l'une quelconque des revendications 1 à 5, dans lequel l'anion alcoxyde est un méthoxyde.

7. Procédé selon l'une quelconque des revendications 1 à 6, dans lequel le rapport du polyester à l'alcoxyde est compris entre 20:1 et 50:1 (p:p).

8. Procédé selon l'une quelconque des revendications 1 à 7, dans lequel le procédé est mis en oeuvre jusqu'à ce qu'un rendement d'au moins 70 % du téréphtalate de formule (I) soit atteint.

9. Procédé selon l'une quelconque des revendications 1 à 8, dans lequel le procédé est mis en oeuvre jusqu'à ce qu'un rendement d'au moins 80 % du téréphtalate de formule (I) soit atteint.

10. Procédé selon l'une quelconque des revendications 1 à 9, dans lequel le procédé est effectué à la pression atmosphérique.

11. Procédé selon l'une quelconque des revendications 1 à 10, dans lequel le polyester est du polyéthylène téréphtalate.

12. Procédé selon l'une quelconque des revendications 1 à 11, dans lequel le procédé comprend en outre une récupération de monoéthylène glycol (MEG).

13. Procédé selon l'une quelconque des revendications 1 à 12, dans lequel le polyester est sous la forme de paillettes de polymère et fait partie d'une charge d'alimentation.

14. Procédé selon la revendication 13, dans lequel la taille moyenne des particules de paillettes de polymère est comprise entre 1 mm et 20 mm.

15. Procédé selon l'une quelconque des revendications 1 à 14, dans lequel le procédé est réalisé à une pression comprise entre la pression atmosphérique et 1379 kPa.
